# EUROPEAN PATENT APPLICATION

(11) **EP 4 631 503 A1**
(43) Date of publication of application: **15.10.2025**
(21) Application number: 24169092.4
(22) Date of filing: 08.04.2024
(51) Int. Cl.: A61K 31/435, A61K 31/505, A61P 35/00

(54) **AVL-292 OR MK-5108 FOR USE IN THE TREATMENT OF PDAC**

(71) Applicant: Molius Saglik Teknolojileri Anonim Sirketi, Ümraniye/Istanbul (TR)
(72) Inventor: MARDINOGLU, Adil, 412 60 Göteborg (SE); YUAN, Meng, 171 72 Solna (SE)
(74) Representative: Kransell & Wennborg KB

(57) **Abstract**

The present disclosure provides AVL-292 or MK-5108 for use in a method of treatment of pancreatic ductal adenocarcinoma (PDAC).

## Description

### TECHNICAL FIELD

The present disclosure relates to the field of treatment of pancreatic ductal adenocarcinoma (PDAC).

### BACKGROUND

Pancreatic ductal adenocarcinoma (PDAC) is the most prevalent type of pancreatic cancer, notorious for its poor prognosis with a five-year survival rate of merely 11%. Its characteristics include diagnosis typically at an advanced stage and a pronounced resistance to conventional chemotherapy.² As such, standard treatments for PDAC, such as surgery and chemotherapy, often fall short for the majority of patients. Despite ongoing research efforts, progress in finding effective treatments for PDAC has been incremental. Metformin emerged as a potential therapy, yet only extended survival by an average of 0.8 months in phase II clinical trials³, underscoring the critical need for more effective drug options for PDAC.

Drug repositioning, compared to traditional drug discovery, is a promising strategy for identifying innovative therapeutic applications for existing medications or drug candidates.⁴ It offers notable advantages in pharmaceutical research, including enhanced efficiency, cost-effectiveness, as well as reduced safety risks during drug development.^{5,6} These benefits have facilitated the successful repurposing of a large amount of approved and preclinical drugs for cancer treatments.⁷⁻¹¹ Thalidomide, for example, has been repurposed to treat multiple myeloma¹² and has shown encouraging results in the treatment of autoimmune illnesses.¹³ Moreover, the surge of multi-omics data and the expansion of public medical databases have paved the way for in silico drug repositioning methods to develop effective treatment strategies. The Cancer Dependency Map (DepMap) is widely regarded as one of the crucial resources for enabling these strategies. As a cell line-deprived database, the DepMap database provides comprehensive estimation and measurement of gene essentiality and drug dependency.

In cancer research, systems biology has been utilized to pinpoint critical dysregulated genes involved in cancer's onset and progression. A prominent technique is constructing gene co-expression networks (GCN), revealing gene interactions across different samples. In conjunction with the network topology analysis, researchers can recognize the highly connected genes, which are likely to function together and potentially affect the cancer progression. The hub genes thus have the potential to offer valuable insights into cancer biology and may serve as potential therapeutic targets.

### SUMMARY

The study presented herein adopts an integrated approach, combining disease-gene prediction with drug screening. First, a survival analysis was performed to link gene expressions with patient outcomes. Function analysis demonstrated that these genes were enriched in pathways associated with cell proliferative signalling, cell metabolic and energetic regulation -aligning with known cancer hallmarks.¹⁴ Next, GCNs were constructed to locate coherent PDAC modules and further identify genes that match the survival signatures. Furthermore, a data-driven drug repositioning method was introduced to pinpoint PDAC treatment candidates. Through drug-gene similarity assessments, it was identified that the drugs AVL-292 and MK-5108 significantly reduced PDAC cell viability.

Hence, the present disclosure provides AVL-292 or MK-5108A for use in a method of treatment of PDAC.

The present disclosure also provides a method of treatment of PDAC in a patient, wherein AVL-292 or MK-5108 is administered to the patient.

### BRIEF DESCRIPTION OF THE FIGURES

Figure 1 shows enriched biological processes in signature prognostic genes (SPGs).
Figure 2 shows mean expression level correlation between PAAD and PACA-AU cohorts.
Figure 3A shows the essential score of identified hub-SPG genes.
Figure 3B shows target genes' expression level (TPM) in normal and tumour tissue deprived from 4 patients.
Figure 4 shows the screened target genes and candidate drugs. Left panel: the correlation coefficients between target genes and corresponding candidate drugs. Right panel: Sensitivity scores of candidate drugs on 12 PDAC cell lines. The score on Pane1 is highlighted by light dots.
Figure 5 relates to drug efficacy on Pane1 and HPAFII. Figure 5A-B show that the compounds AVL-292 and MK-5108 significantly suppressed the cell proliferation on both Pane1 and HPAF II. Figure 5C-D shows that AVL-292 causes notable inhibition on cell invasion on both Pane1 and HPAF II at nontoxic dosage and that MK-5108 and AVL-292 can significantly reduce the cell invasion on Pane1.
Figure 6A shows the effect of 10 µM of AVL-292 or MK-5108 on cell viability in an MTT assay on Pane1.
Figure 6B shows western blots quantifying how AVL-292 and MK-5108 (at 10 µM) affected the target genes when decreasing the cell viability of Pane1 cells.
Figure 6C shows a TF-target gene regulatory network of MK-5108 in PAAD M124. The CDCA5 is co-expressed with AURKA and AURKB, which are the drug targets of MK-5108, and regulated by multiple TFs.

### DETAILED DESCRIPTION

Pancreatic ductal adenocarcinoma (PDAC) is characterized by its high level of aggressiveness, poor prognosis and limited treatment options. Despite multiple chemotherapy regimens for the treatment of PDAC, their effectiveness is constrained by the significant drug resistance of the tumour. Hence, it is imperative to identify novel pharmaceutical targets and develop drugs for PDAC treatment. In this context, drug repurposing has emerged as a promising approach for drug discovery, as it allows for the identification of new therapeutic uses for existing drugs with established safety profiles¹². The study described herein takes a drug repositioning approach aimed at identifying potential therapeutic targets and effective drugs for PDAC treatment.

The study employed two independent PDAC cohorts to identify 314 prognostic genes, despite of the presence geographical differences across the cohorts. In previous research of 17 major cancer types²¹, a general pattern that the genes associated with poor prognosis were enriched in cell growth progress was observed. The current PDAC study also demonstrated a consistent tendency. Nearly half of the PDAC SPGs had an unfavourable association with patient survival outcomes, where the unfavourable SPGs were reported to be enriched in cycle-cell processes and regulation, especially in chromosome separation involved in mitosis.

The PDAC-specific GCNs were constructed to reveal the essential gene modules involved in PDAC progress. The dysregulated modules in PDAC indicated that multiple key pathways, such as cell cycle regulation, DNA damage response and immunological processes, were significantly maladjusted. These dysregulations may have a negative impact on the overall survival outcome. Based on these findings, possible pharmaceutical agents that can suppress the proliferation of PDAC cells were discovered.

AVL-292, as one of the repurposed drugs for PDAC treatment in this study, is a small molecular inhibitor that targets Bruton's tyrosine kinase (BTK). BTK is a key regulator of B-cell receptor signalling, development, differentiation and survival. Especially for PDAC, BTK could suppress cancer cell growth and improve chemotherapy responsiveness through the meditation of BTK signaling.³⁵

MK-5108, another repurposed drug in this study, emerged as the most promising compound repurposed for the therapeutic intervention of PDAC, as it showed the strongest inhibition to Pane1 and HPAF II cell viability. MK-5108 is a selective aurora kinase inhibitor designed to target *AURKA* by the disruption of mitotic AURKA Thr288 phosphorylation, resulting in the induction of mitotic exit delay.³⁴ It was identified that *AURKA* is one of the hub genes in PAAD prognostic modules (M124). Additionally, *AURKA* has been observed to be co-expressed with all four PDAC target genes. Moreover, *AURKA* is also identified to be co-expressed with *CDCA5, TPX2* and several kinase family members in multiple cancer cases.^{36,37} In line with these findings, MK-5108 significantly increased the AURKA level in Pane1 and increased the protein level of *CDCA5, KIF18A, KIF23* and *TPX2.* A significant increase in apoptosis was also observed, suggesting that MK5108 may inhibit a shared transcription factor involved in apoptosis and subsequently impact several proteins associated with apoptosis. Other studies have confirmed that the inhibitory function of AURKA is accomplished through a substantial reduction in AURKA autophosphorylation, rather than by impacting the expression level of AURKA.^{38,39} Moreover, MK-5108 is well tolerated by patients when taken orally, according to a phase I trial.¹

In conclusion, the present study highlights the effectiveness of drug repurposing as a method to find treatments for PDAC. The utilization of GCN analysis and the integration of gene perturbation expression profiles deprived of heterogeneous data sources represents a powerful approach for the discovery of drug targets and repurposing of drugs. In summary, the study emphasizes the value of drug repurposing as a feasible and economically prudent approach to develop efficacious PDAC treatments. The integration of GCN analysis with gene perturbation expression profiles, derived from a range of heterogeneous data sources, has proven to be a powerful method for the discovery of novel drug targets and the repurposing of the existing drugs AVL-292 and MK-5108.

Accordingly, a first aspect of the present disclosure provides AVL-292 or MK-5108 for use in a method of treatment of pancreatic ductal adenocarcinoma (PDAC).

The method of the first aspect preferably comprises oral administration of the drug (AVL-292 or MK-5108).

A second aspect of the present disclosure provides a method of treatment of pancreatic ductal adenocarcinoma (PDAC) in a patient, wherein AVL-292 or MK-5108 is administered to the patient.

The administration of the second aspect is typically oral administration.

### EXAMPLES

### Materials and methods

### Data source and pre-processing

The RNA sequencing data of the PDAC cohort was retrieved from The Cancer Genome Atlas 15 (TCGA, project PAAD-TCGA). The raw data fastq files were downloaded through the GDC client. The transcript-level read counts were quantified by Kallisto¹⁶ with GENCODE annotation of human reference transcriptome (GRCh38, release 103). All pancreatic adenocarcinoma samples were screened and 176 donors with both RNA-seq data deprived from primary tumour solid tissue samples and well-recorded clinical information were kept.

The gene expression data (transcripts per million, TPM) and patient clinical details from the Australian cohort (PACA-AU) were sourced from the International Cancer Genome Consortium (ICGC, accessed on 4 June 2022 at http://icgc.org/). 80 individuals with primary tumour solid tissue samples and accompanying clinical information were selected. For patients with multiple tumour samples, the sample with the highest cellularity was chosen for final sequencing data. TPM values were derived from the raw data originally presented in FPKM (fragments per kilobase of exon per million reads mapped) format.

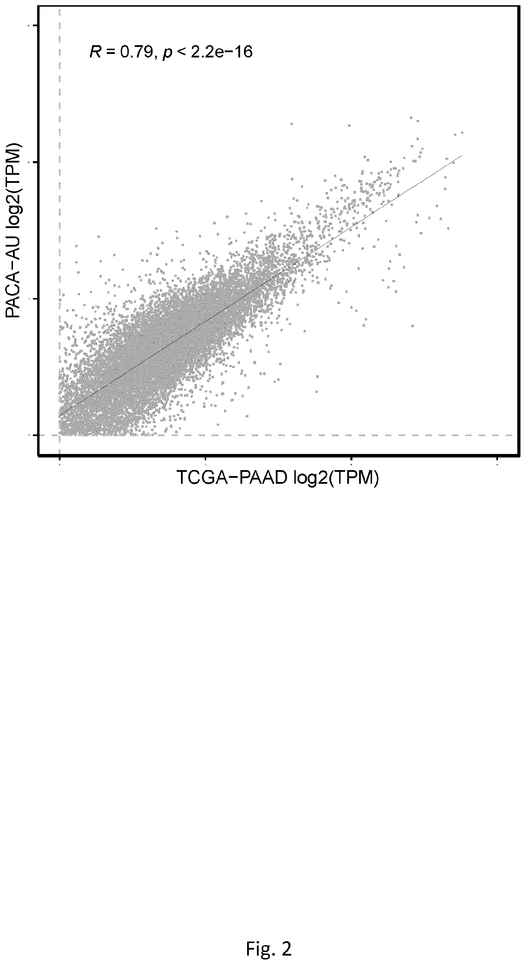

In the analysis of the two cohorts, protein-coding genes with an average TPM greater than 1 were considered actively expressed genes and were retained for subsequent analysis to reduce the noise from lowly expressed genes. The correlation between gene expression levels in the PAAD and PACA-AU cohorts was assessed using Spearman correlation (Figure 2), including only genes actively expressed in both cohorts. The statistical analysis was conducted using RStudio (R version 4.1.0).

### Survival analysis

Both univariable Cox regression model and the Kaplan-Meier (KM) survival analysis were conducted to evaluate the association between the patients' transcriptomic profiles and clinical survival outcomes, with the time from enrolment to overall death (survival time less or equal to 0 day excluded). For univariable Cox analysis, the hazard ratio of each gene was calculated to investigate the association between gene expression and patients' days alive. Genes with a p-value of less than 0.05 were kept as significant genes related to survival outcomes. For KM analysis, patients were stratified into high and low subgroups based on their gene expression levels. The optimal TPM cut-offs for these groups were determined using all TPM values ranging from the 20th to 80th percentiles to divide the patients. The log-rank tests were used to estimate the group differences in the survival outcomes among the groups. The value yields with the lowest p-value were selected. Genes with p value less than 0.05 were identified as prognostic genes. In addition, if the patients with high expression of a selected prognostic gene had a higher observed survival time compared to the expected event, the gene was classified as a favourable prognostic gene; otherwise, it is defined as an unfavourable prognostic gene. The analyses were conducted using R with the package "survival".

Genes with p-values lower than 0.05 in both survival analyses were extracted. Among these genes, those that demonstrated consistent prognostic implication (either favourable or unfavourable) in both Cox analysis and KM analysis across two cohorts were identified as signature prognostic genes (SPGs) and adopted for further analysis.

### Functional enrichment analysis

The R package "clusterProfiler" was used to identify the enriched biological progress in SPGs. The gene ontology (GO) functional enrichment analysis was performed by the enrichGO function (p < 0.05).

### Co-expression analysis and module identification

Spearman correlation coefficients of gene expression level (between any two genes) were calculated to estimate the gene co-expression association. Only the correlations ranked in the top 1% among all gene pairs were retained for the construction of GCN. The Walktrap algorithm, a method capable of detecting densely connected modules by leveraging structural similarity, was performed to find gene modules with high transitivity in GCN. Only modules that contain more than 20 nodes and have connectivity coefficients larger than 0.5 were extracted for further analysis.

Modules with no statistical overlap with SPGs were then classified as nonprognostic modules. Subsequently, prognostic module pairs that have significant overlap with SPGs among the PAAD and PACA-AU cohorts were identified. Two prognostic modules, one deprived from the PAAD cohort and the other from the PACA-AU cohort, had a significant overlap (hypergeometric test, p < 0.05) and were identified as the most promising gene module pair in PDAC, as they showed similar network gene components across two distinct cohorts. All analysis was conducted on RStudio, network visualization was carried out using Cytoscape¹⁷.

### Concordance analysis

As previously described^{18,19}, the accumulative hypergeometric model was employed to assess the statistical significance of the overlap between two sets of prognostic genes. This model was also used to see if the genes inside a module had any significant overlap with the prognostic genes. The Jaccard Coefficient (JC) was calculated by dividing the size of the overlapped genes by the size of the union of two lists of genes.

### Identification of PDAC Target Genes

The topology analysis was conducted to identify the hub genes that influence the prognostic module networks. Among the overlapped prognostic module pair, three network topological parameters (degree, betweenness and closeness) of all involved genes were calculated. The genes were then arranged in decreasing order inside each module. Taking degree as an illustrative example, the genes with degree values ranking higher than 20 per cent in each module were extracted. The degreehub genes shared for this module pair were then identified as they were shared by both modules. Similar steps were used to obtain betweenness-hub genes and closeness-hub genes. Subsequently, all three shared hub gene lists were considered and the overlapped genes were regarded as the most potential candidate genes for PDAC drug targets.

The essential scores of genes in 16 primary PDAC cell lines were downloaded from the DepMap portal (https://depmap.org/portal/, 04 January 2022). These scores are estimated based on the CRISPR-Cas9 essentiality screens. A lower gene score indicates this gene exerts stronger effects on tumour cell proliferation after CRISPR-Cas9 knockout of this gene. Typically, a gene with an essential score lower than -0.5 means that the cell will grow slower when the gene is knocked out²⁰. Only genes with an essential score less than -0.5 were reserved as most potential target genes for PDAC.

The immunohistochemistry (IHC) staining images of normal and tumor liver cells were downloaded from the Human Protein Atlas (https://www.proteinatlas.org/). The generation of IHC and identification of staining intensity was described in their previous research²¹. For each target gene, only the images generated by the same antibody were selected for fair comparison.

### Transcription factors target gene co-occurrence network

The over-represented transcription factors (TFs) were annotated by Enrichr ²² based on the TF-Gene co-occurrence database. The top 5 TFs ranked by p-value in both the PAAD and PACA-AU cohort were extracted. To further explore the interaction between the TFs-target genes, a co-occurrence network was constructed by examining the String ²³ database.

### Drug Repositioning for PDAC

The primary PRISM Repurposing dataset comprises the results of pooledcell line chemical-perturbation viability screens. A total of 4,518 compounds were evaluated against 578 cell lines. Additionally, the establishment of CRISPR knockout profiles has facilitated systematic investigations into the effects of genes on cell viability.

The pancreatic cell lines' count expression profiles and gene dependency files were downloaded from the DepMap Public (version 22Q1). The drug sensitivity primary screen profiles were obtained from the DepMap (PRISM Repurposing 19Q4). To screen the available cell lines with well-recorded information, 12 primary PDAC cell lines were kept for further analysis since they were tested by all three profiles as well as have fewer than 15% NA treatment values.

The hypothesis posited that drugs capable of inhibiting cancer cell growth would yield comparable effects with target gene knockout on gene expression (count) changes. Genes with a count value larger than 10 were retained for further analysis. Three steps were carried out to screen the candidate compounds. First, the Spearman correlation matrix between the profiles and target gene essential score (ES-matrix) and drug sensitivity score (DS-matrix), respectively, was calculated. Next, the correlation matrix between ES- and DS-matrix, namely corr2matrix, was generated to estimate the effect association between each target gene and drug compounds. At last, the corr2matrix was refined and the top 3 drugs for each target gene was identified as potential candidate drugs for PDAC. For each target gene, the gene-drug pairs with correlation > 0.6 were considered as having significantly comparable effects on inhibiting cell growth.

### Cell culture, siRNA transfection, drug treatment, and cell viability assay

Pane1 and HPAF II cells were cultured with DMEM (D0822, Sigma-Aldrich) supplemented with 10% fetal bovine serum (FBS, F7524, Sigma-Aldrich), 1% P/S (P4333, Sigma-Aldrich). For siRNA treatment, 200,000 cells for a 6well plate and 20,000 cells per well for a 96well plate were seeded. siRNAs were purchased in Origene, CDCA5 (SR314367, Origene), KIF18A (SR313129, Origene), KIF23 (SR306300, Origene), TPX2 (SR307905, Origene), and cells were transfected by Lipofectamine^{®} RNAiMAX (13778-075, Invitrogen) for two days. Small molecules which repositioned were purchased by AVL-292 (HY-18012, MCE), BCI-50 (HY-17553, MCE), MK-5108 (HY-13252, MCE), AZD1208 (HY-15604, MCE), SKA-31 (HY111655, MCE), Y-26763 (HY-101069, MCE), Estropipate (HY-B1361, MCE), Tiquizium (sc-474386, Santa Cruz), Acyclovir (HY-17422, MCE), and Cyproterone acetate (HY-13604, MCE) and dissolved into DMSO. Drugs were treated to cells for two days at 10µM. Cell viability was measured by MTT assay (M6494, ThermoFisher) by following the manufacturer's instructions. O.D values were detected with a microplate reader (Hidex Sense Beta Plus).

### Western blots

Pane1 and HPAF II cells were seeded into a 6-well plate at 200,000 cells per well concentration. After siRNA transfection and drug treatment, whole cell lysate was prepared with CelLytic M (C2978, Sigma-Aldrich) buffer and samples were prepared with 2x Laemmli Sample Buffer (1610737, Biorad) at 20µg protein lysate. SDS PAGE was performed Mini-PROTEAN^{®} TGX^{™} Precast Gels (Bio-Rad) and transferred using Trans-Blot^{®} Turbo^{™} Transfer System (Bio-Rad). CDCA5 (ab192237, abcam), KIF18A (ab72417, abcam), KIF23 (HPA068831, Sigma Aldrich), TPX2 (ab252944, abeam), Aurora A (ab108353, abeam), Aurora B (ab287960, abcam), PARP (9542S, Cell signaling), Active Cas9 (ab2324, abcam), β-actin (ab8227, abeam), GAPDH (ab8245, abeam), and α-tubulin (ab7291, abcam) were blotted as a primary antibody for overnight. Secondary antibodies, Goat Anti-Rabbit HRP (ab205718) and goat anti-mouse IgG-HRP (sc2005, Santa Cruz Biotechnology, Inc.) were blotted for one hour. The protein band was detected with ImageQuantTMLAS 500 (29-0050-63, GE).

### Invasion assay

Panc1 (20,000 cells per transwell), and HPAF II (80,000 cells per transwell) were transferred into the upper well of collagen-coated 8µm pore transwell insert (3422, CORNING) with serum-free media treated with non-toxic drug concentration. Transwells were placed into culture media in a 24-well plate, then growth factors in growth media induced invasion via chemo attraction and incubated in 37°C CO₂ incubator for one day. Cells were fixed with 3.7 % formaldehyde for 30 minutes at room temperature and washed with PBS twice. Cells were stained with 1% crystal violet in PBS for 20 minutes and residual cells in the inner membrane were swabbed gently with a cotton swab. Invaded cells located outer membrane were imaged by ZOE Fluorescent Cell Imager (Bio-rad, USA) and counted by image.

### TF-gene regulatory network

The drug targets were obtained from DepMap Public (version 22Q1). TF-gene interaction information was deprived from the Trrust ²⁴ database. Combining the co-expression relationships calculated in PDAC GCNs, module-wised TF-target gene regulatory networks were established for PAAD and PACA-AU cohorts, and further visualized in Cytoscape ¹⁷.

### Results

### Survival Analysis Identifies Signature Prognostic Genes of PDAC

Two independent cohorts were employed to identify robust SPGs for PDAC. The PAAD cohort consisted of 176 patients with a mean age of 65 years old (average age of survivors: 64 years old, and average age of deceased: 66 years). In contrast, the PACA-AU cohort had an average age of 68 years, with surviving patients averaging 67 years and deceased patients averaging 69 years. Univariable Cox regression and Kaplan-Meier analysis were performed to investigate the correlation between the expression levels of each protein-coding gene and patients' survival outcomes in both PAAD and PACA-AU cohorts. A strong correlation (r=0.79, Figure 2) in the expression levels of protein-coding genes between the two cohorts was observed, suggesting a similarity in the gene expression patterns across both cohorts. Furthermore, the prognostic values of these genes were estimated in two cohorts, respectively. In the PAAD cohort, Cox and KM analyses identified 4,128 and 8,166 prognostic genes with p-values lower than 0.05, respectively. Among these, 4,040 genes were found to be common to both analyses (p<0.05, hypergeometric test). Similarly, in the PACA-AU cohort, 1,600 and 6,016 prognostic genes were identified as associated with survival outcomes by Cox and KM analyses, respectively, with 1,502 genes being shared between the two analyses (p<0.05, hypergeometric test).

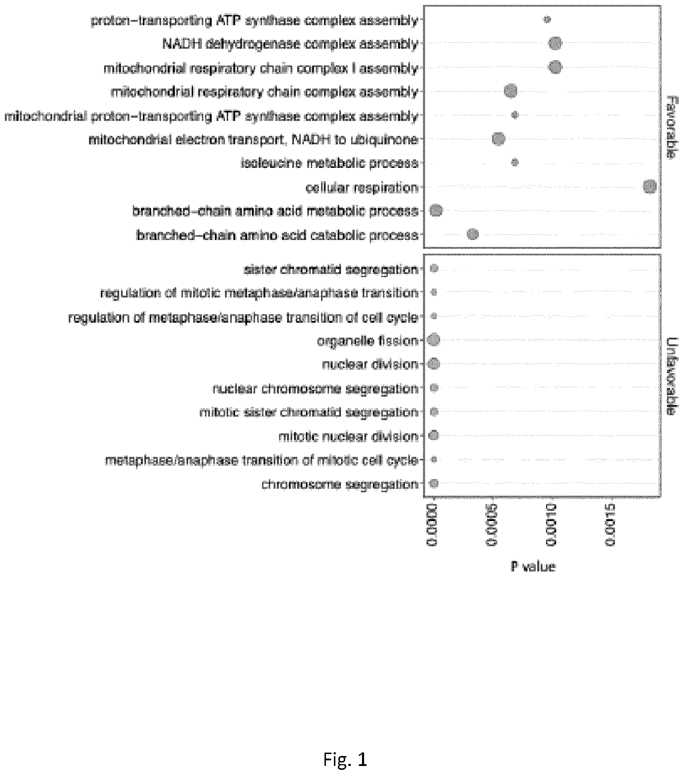

In addition, 314 SPGs that were consistent across both PAAD and PACA-AU cohort analyses were identified (Table S1). These SPGs were almost evenly distributed, with 152 classified as favourable and 162 as unfavourable. The favourable SPGs, characterized by high expression levels associated with improved survival outcomes, were predominantly involved in respiratory-related processes and ATP-synthase assembly activities (Figure 1). Conversely, the unfavourable SPGs, linked to poorer patient survival, were implicated in various phases of cell mitosis.

### Co-expression Network Analysis Identifies Hub Modules of PDAC

GCN was constructed for both PAAD and PACA-AU cohorts independently. In brief, the Spearman correlation coefficient was calculated for each pair of genes based on their mRNA expression profiles. The gene pairs with correlation ranking higher than 1% were extracted for the construction of a co-expression network. For the PAAD cohort, this process resulted in a GCN with 1,999,866 gene-gene links between 4,674 genes, with a correlation ranging from 0.69 to 0.99. Within this network, 10 modules were identified, each containing more than 20 genes and have connectivity coefficients higher than 0.5. Similarly, for the GCN of the PACA-AU cohort, a total of 1,812,488 gene-to-gene links were retrieved, encompassing 2,231 genes and having a Spearman correlation ranging from 0.60 to 0.99. Subsequently, 15 modules were then obtained with the same manner.

Next, the SPGs were superimposed to all modules to conduct a concordance analysis. In the GCN of PAAD, two modules (M21 and M54) were observed to have significant overlaps with favourable SPGs (hypergeometric test, p<0.05). Consequently, these two modules were denoted as favourable modules. Conversely, two modules (M60 and M124) showed significant overlaps with unfavourable SPGs and were thus identified as unfavourable modules. Similarly, in PACA-AU modules, M1 and M97 were categorized as favourable modules, while M45, M50 and M93 were designated as unfavourable modules (hypergeometric test, p<0.05).

GO term enrichment analysis was performed on these gene modules for the two GCNs to investigate their functional features (Table S2-3). It is noteworthy that most of the modules can be categorized into five groups based on the parental biological processes their enriched GO terms are associated with. Interestingly, the biological processes were conserved across both GCNs. For instance, M18, M20, M64, M98 and M133 in PAAD GCN were found to be related to metabolic processes, including lipid catabolism, protein transportation and protein secretion. The aforementioned processes were also associated with M1, M76, M83, M90 and M94 identified in the GCN of PACA-AU. Moreover, RNA processing-related terms were enriched on modules M16 and M21 of PAAD GCN, and the same biological processes were enriched in M7 and M97 in the PACA-AU GCN. In addition, it was found that mitosis processes were related to the unfavourable modules in both GCNs. Especially, the unfavourable gene module M124 in PAAD GCN and M45 in PACA-AU GCN were enriched with genes related to cell cycle regulation and increased cell proliferation in the tumour, which is a well-known hallmark of cancer.²⁵ Immune responses were also found to be associated with unfavourable modules, including M60 in PAAD and M93 in PACA-AU, suggesting the enhancement of immune processes in the progression of PDAC pathology.

Moreover, pair-wised comparisons were conducted across all prognostic modules to identify conserved prognostic gene modules between these two GCNs (Table S4). Hypergeometric tests were performed on the prognostic module pairs and the JC for all module pairs was calculated to quantify their concordance. The analysis yielded significant findings in both favourable and unfavourable modules. Specifically, two favourable modules, M21 from the PAAD GCN and M97 from the PACA-AU GCN showed significant overlap (JC = 0.23, p<0.001, hypergeometric test). As both were enriched with genes involved in the RNA splicing process, it indicates that increased RNA splicing is a prevalent process in PDAC. In the case of unfavourable modules, the M60 (PAAD) and M93 (PACA-AU) pair, as well as the M124 (PAAD) and M45 (PACA-AU) pair, demonstrated statistically significant overlaps (JC=0.24 and JC=0.59, respectively, both with p<0.001). These similarities in functional modules likely represent fundamental and essential mechanisms associated with PDAC.

### Identification of Target Genes in PDAC

To ascertain the structure of the consensus prognostic gene modules between the two GCNs, the topological analysis was performed to estimate the centrality of genes inside the overlapped module pairs. Among the three prognostic module pairs, the M45-M124 module pair exhibits the highest topology similarity concerning network degree (r=0.64), betweenness (r=0.59) and closeness (r=0.63). Additionally, all genes were sorted in the overlapped module pairs in descending order according to each centrality parameter within each module. With each module, the genes that ranked in the top 20% based on each topology parameter were extracted, and the hub genes were thus noted as the shared top genes across all three parameters. Consequently, no shared hub gene was found for favourable module pair M21-M97. As for the unfavourable module pair M60-M93, the gene *IFIT3,* namely interferon-induced protein with tetratricopeptide repeats 3, was found and shown as a top gene as well as one of the SPGs. The *IFIT3* is a potential tumour-promoting gene and its over-expression can lead to an increase of tumour size.²⁶ Notably, in the unfavourable module pair of M124-M45, the hub genes showed higher similarity. The module M124, encompassing 182 genes, was subjected to a detailed analysis, from which the top 20% of genes were extracted based on degree, betweenness, and closeness parameters. This process identified 35 genes as the highest ranking in each category. Among these, 24 genes were consistently present in all three top lists, thereby designating them as the hub genes of M124. Similarly, the module M45, comprising 224 genes, led to the identification of 45 top-ranked genes in each of the three categories. Of these, 30 genes were common across all categories, qualifying them as hub genes for M45. Notably, nine genes emerged as shared hub genes between both modules M124 and M45. Four of these shared genes - *KIF23, KIF18A, TPX2,* and *CDCA5* - had already been identified as SPGs in the survival analysis.

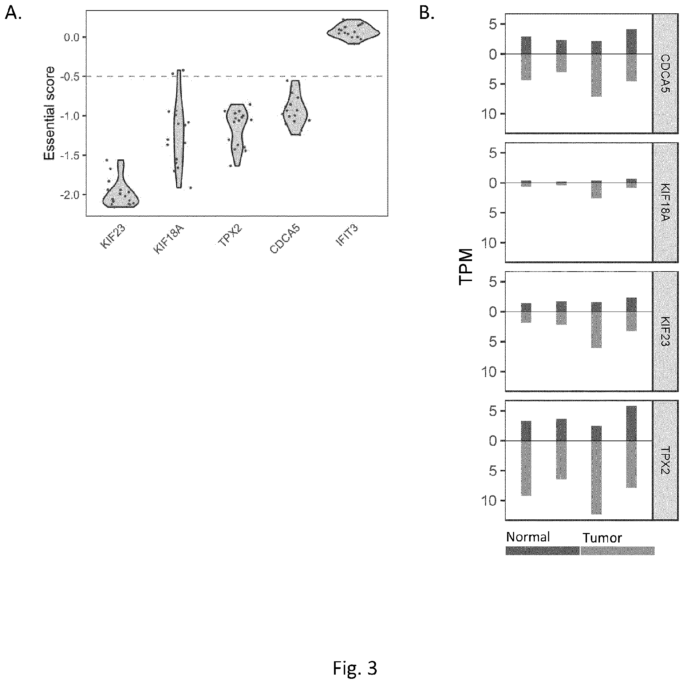

In summary, 5 genes designated as shared-SPGs were identified, which means they are hub genes within module networks and associated with poor survival outcomes in both PADC cohorts. Subsequently, essential scores from the DepMap data portal ²⁷ with 16 primary pancreatic cancer cell lines were obtained. The essential scores were estimated by the genome-scale CRISPR-Cas9 viability screens in various cell lines to identify specific genetic dependencies. A lower essential score indicates a higher influence on tumour cell proliferation and DNA replication. In this context, the essential scores of the five shared-SPGs were examined. Four genes (*KIF₂₃, KIF18A, TPX2* and *CDCA₅*) emerged as potential target genes, given their essential scores were less than -0.5 (Figure 3A). As expected, these four genes showed a consistent unfavourable association with the survival of the PDAC patients.

Also the mRNA expression profiles of PAAD normal and adjacent tumour tissues were evaluated to access the expression levels of the 4 target genes. As shown in Figure 3B and Table S5, all 4 target genes showed more than 2-fold higher expression in the tumour tissue compared to the adjoining normal tissue. This is also supported by the antibody-based immunohistochemistry image results, where most of the target proteins, namely CDCA5 (with antibody HPA076007), KIF18A (with antibody HPA039484) and TPX2 (with antibody HPA005487), exhibited lower staining intensity levels in normal Exocrine glandular cells compared to the tumour cells. As these target genes encode mitotic-related processes and are involved in cellular division processes, this altered expression in the tumour potentially indicates elevated tumorigenesis or tumour progression.

In addition to the notable upregulation of the target genes in tumour tissue, similar regulatory mechanisms among the target genes were also observed. The top 5 over-represented TFs in M124 and M45, respectively, were extracted (Table S6). Among the two TF lists, 4 shared TFs (*DEPDC1, WDHD1, KNTC1* and *TCF19*) were found to co-regulate with 4 target genes and were also involved in mitosis processes including DNA replication, chromatin assembly and segregation. Notable, *TCF19* encodes proteins involved in the cell proliferation and apoptosis of pancreatic beta cells. ²⁸

### Drug repositioning for PDAC

In the study, a profile-based drug repositioning approach was employed, integrating transcriptional responses from CRISPR Cas9 gene knockout experiments with chemical perturbation data sourced from the DepMap database. This approach was aimed at identifying potential drug-specific genes implicated in pancreatic cancer.

Initially, the expression profiles of pancreatic cancer cell lines were analyzed, considering genes with a mean count value above 10 as actively expressed (13,957 genes) for subsequent analysis. Two matrices were established: the Essential Score matrix (ES-matrix) representing the cruciality of target genes for cell survival, and the Drug Sensitivity matrix (DS-matrix), illustrating how gene expression correlated with drug sensitivity. All four target genes under investigation were incorporated into the ES-matrix.

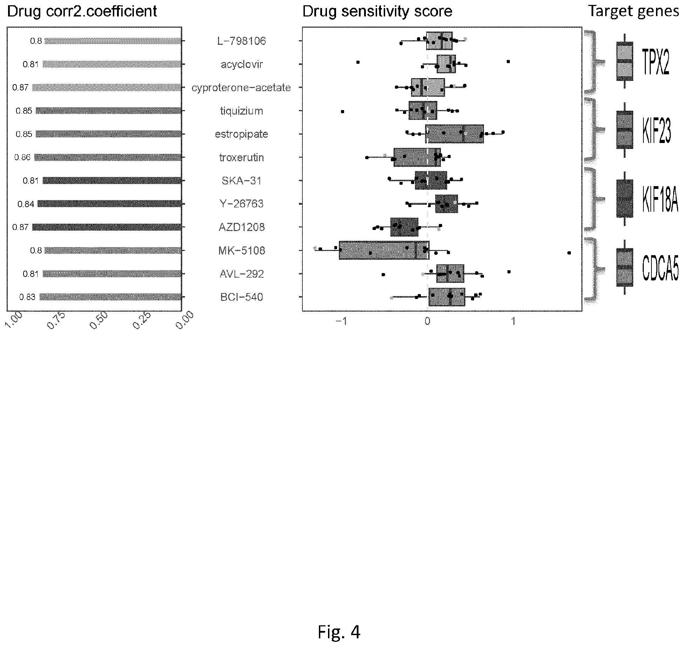

The DS-matrix consisted of 4,868 chemical compounds, each tested across various concentrations. Then a correlation matrix (corr2matrix) between the ES- and DS-matrices was calculated (Table S7) to assess the relationship between each target gene and the array of drug compounds. This analysis allowed us to rank the drugs based on their correlation coefficients with the target genes. Higher coefficient values indicated a stronger similarity between the drug's action and the genetic impact observed from the gene knockout. Ultimately, through optimization of the corr2matrix, the three most promising drugs for each target gene were identified as potential therapeutic candidates for pancreatic cancer (Figure 4, left panel). PDAC is widely recognized for its significant resistance to pharmacological therapies. The drug candidates' sensitivity on selected 12 PDAC cell lines were thus examined. Among 12 candidate compounds, 5 of them have a sensitivity score lower than 0 on the Pane1 cell line (Figure 4, right panel), which means the corresponding treatments are more likely to affect cell viability. A comprehensive literature review on these five drugs was then conducted. BCI-540, also known as Coluracetam, is a glutamate drug developed to treat anxiety and depression ²⁹. AVL-292 and MK-5108 are inhibitors that have been investigated in various research for cancer treatments or research ^{30,31}. SKA-31 as a Ka3.1 activator, has also been discussed in the context of cancer ³². Troxerutin exhibits an anti-cancer effect on cell line model³³. Based on these findings, these five drugs have been selected for further experimental validation, building upon the insights gained from the current results.

### In vitro validation of candidate drugs

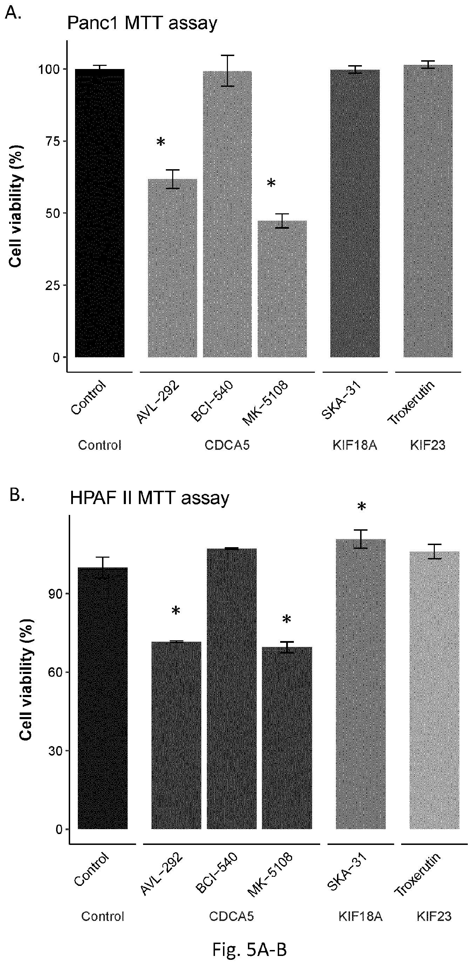

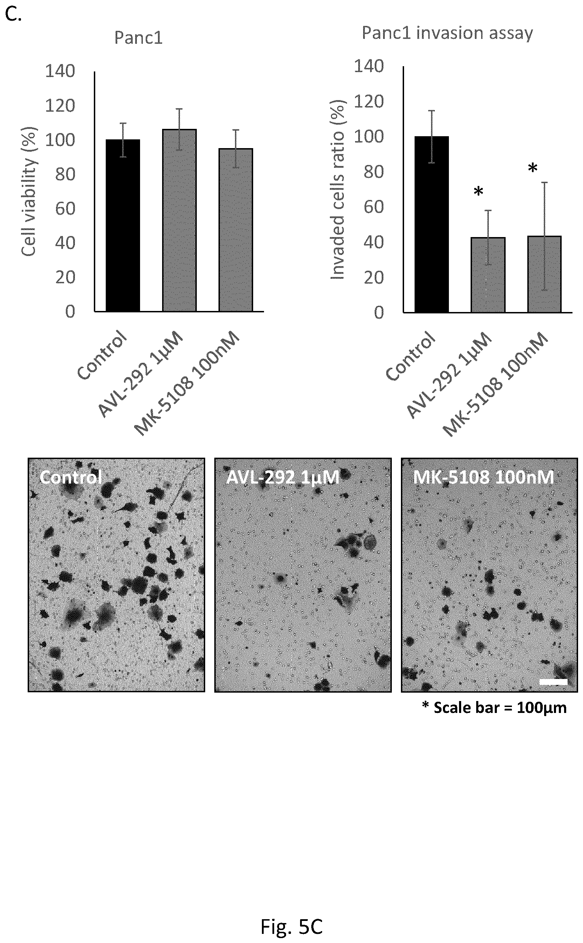

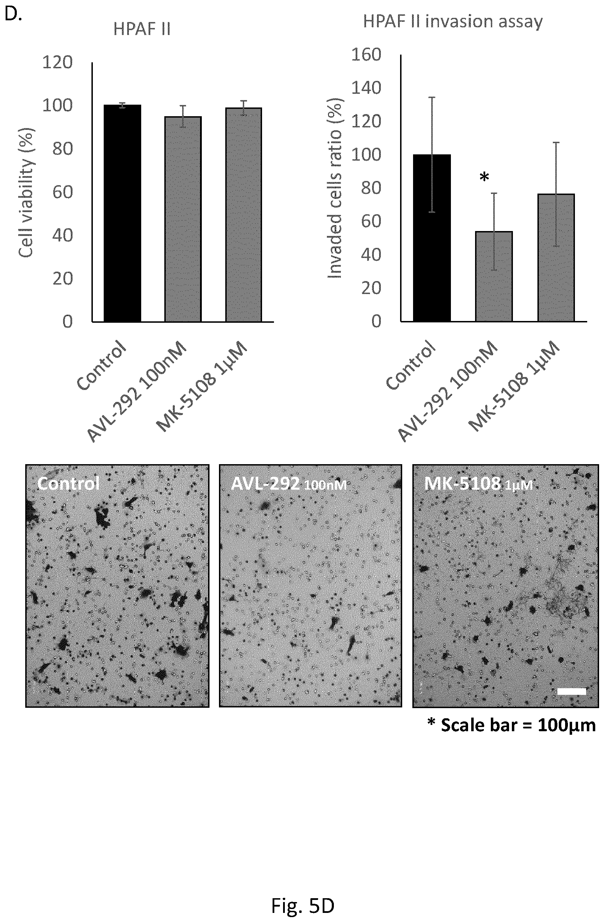

To further investigate the anti-cancer drug efficacy, Pane1 cells were treated with 10µM of compounds for two days (Figure 5A-B). In both cell lines, treatment with 10µM AVL-292 and MK-5108 resulted in a notable reduction in cell viability. AVL-292 demonstrated the viability of 60.7%±3.3 for Pane1 and 71.6%±0.35 for HPAF II, while MK-5108 decreased the viability of 45.8%±2.49 for Pane1 and 69.5%±2.06 for HPAF II. Next, the non-toxic concentrations of AVL-292 and MK-5108 for invasion assay were investigated. As shown in the left panel of Figure 5C-D, AVL-292 exhibited non-toxicity at 1µM for Pane1 and 100nM for HPAF II, while MK-5108 showed non-toxicity at 100nM and 1µM for Pane1 and HPAF II, respectively. These concentrations of the compounds significantly reduced cancer cell invasiveness in Pane1 cells by AVL-292 (42.7% ±15.4) and MK-5108 (43.37% ±30.6). In HPAF II cells only AVL-292 (53.9%±23.0) showed significantly decreased invasiveness.

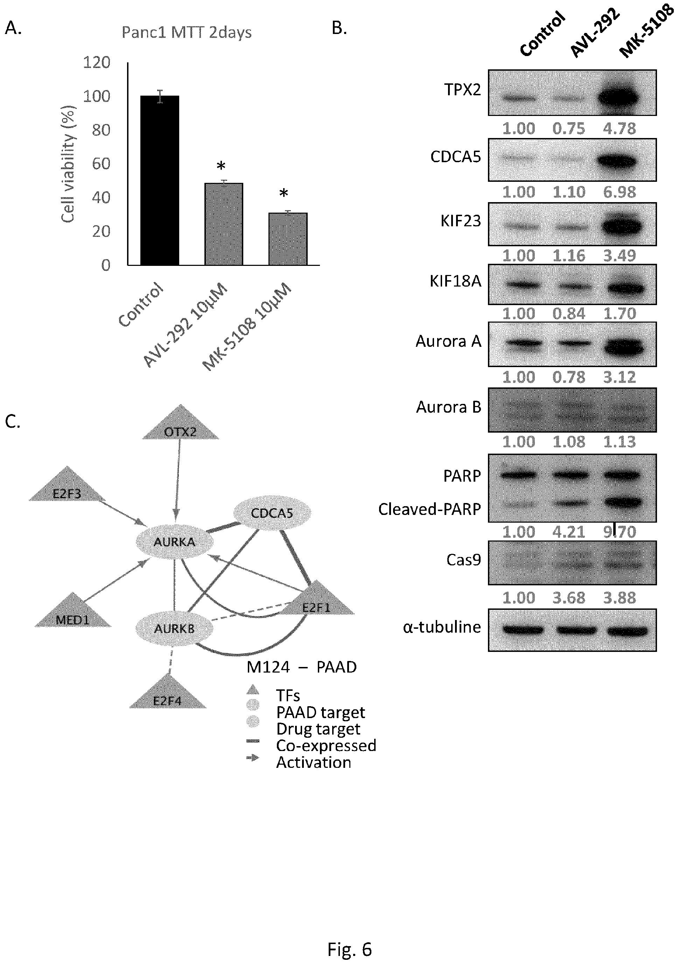

Moreover, it was investigated how AVL-292 and MK-5108 affect the target genes when decreasing the cell viability of Pane1 cells (Concentrations can be found in Figure 6A). With the treatment of AVL-292, TPX2 and KIF18A showed decreased expression levels to 75% and 84% compared to control, while CDCA5 and KIF23 showed slight increases to 110% and 116%, respectively (Figure 6B).

For MK-5108 specifically, *AURKA* and *AURKB's* expression changes have also been investigated since MK-5108 is a designed selective aurora inhibitor targeting to these two genes. The TF regulatory network was constructed to show the regulatory of CDCA5 within M124, combined with the co-expression correlations based on PAAD GCN (Figure 6C). *AURKA* and CDCA5 are co-expressed and coregulated by E2F1, a crucial cell cycle regulator in cell proliferation. Additionally, *AURKA* was also identified as a hub gene in M124-PAAD. Western blot analysis showed MK-5108 over-expressed target genes (*TPX2, CDCA5, KIF23,* and *KIF18A*)*,* as well as *AURKA* (Figure 6B), but no obvious effect was found on *AURKB.*

Notably, both repositioned drugs AVL-292 and MK-5108 increased the protein expression level of cleaved PRAP and Cas9 which are apoptotic genes. Significant cell viability descension with target gene knockdown was not observed. It may indicate that drugs are not affecting apoptosis by single gene knockdown. Research has demonstrated that aurora inhibitors could induce mitosis delay in cancer cells³⁴, potentially serving as a trigger for the initiation of apoptosis. However, more analysis and experiments are warranted to thoroughly explore the specific mechanisms underlying this observed effect.

### REFERENCES

1 Amin, M. et al. A phase I study of MK-5108, an oral aurora a kinase inhibitor, administered both as monotherapy and in combination with docetaxel, in patients with advanced or refractory solid tumors. Invest New Drugs 34, 84-95 (2016).
2 Oberstein, P. E. & Olive, K. P. Pancreatic cancer: why is it so hard to treat? TherapAdv Gastroenterol 6, 321-337 (2013).
3 Kordes, S. et al. Metformin in patients with advanced pancreatic cancer: a double-blind, randomised, placebo-controlled phase 2 trial. The Lancet Oncology 16, 839-847 (2015).
4 Ashburn, T. T. & Thor, K. B. Drug repositioning: identifying and developing new uses for existing drugs. Nat Rev Drug Discov 3, 673-683 (2004).
5 Parvathaneni, V., Kulkarni, N. S., Muth, A. & Gupta, V. Drug repurposing: a promising tool to accelerate the drug discovery process. Drug Discov Today 24, 2076-2085 (2019).
6 Pushpakom, S. et al. Drug repurposing: progress, challenges and recommendations. Nat Rev Drug Discov 18, 41-58 (2019).
7 Fu, L. et al. Repurposing non-oncology small-molecule drugs to improve cancer therapy: Current situation and future directions. Acta Pharm Sin B 12, 532-557 (2022).
8 Gonzalez-Fierro, A. & Duenas-Gonzalez, A. Drug repurposing for cancer therapy, easier said than done. Semin Cancer Biol 68, 123-131 (2021).
9 Lu, C., Li, X., Ren, Y. & Zhang, X. Disulfiram: a novel repurposed drug for cancer therapy. Cancer Chemother Pharmacol 87, 159-172 (2021).
10 Cavalla, D. & Singal, C. Retrospective clinical analysis for drug rescue: for new indications or stratified patient groups. Drug Discovery Today 17, 104-109 (2012).
11 Liu, Z. et al. In silico drug repositioning - what we need to know. Drug Discovery Today 18, 110-115 (2013).
12 Pushpakom, S. et al. Drug repurposing: progress, challenges and recommendations. Nature Reviews Drug Discovery 18, 41-58 (2019).
13 Millrine, D. & Kishimoto, T. A Brighter Side to Thalidomide: Its Potential Use in Immunological Disorders. Trends in Molecular Medicine 23, 348-361 (2017).
14 Hanahan, D. Hallmarks of Cancer: New Dimensions. Cancer Discovery 12, 31-46 (2022).
15 Cancer Genome Atlas Research, N. et al. The Cancer Genome Atlas Pan-Cancer analysis project. Nat Genet 45, 1113-1120 (2013).
16 Bray, N. L., Pimentel, H., Melsted, P. & Pachter, L. Near-optimal probabilistic RNA-seq quantification. Nature Biotechnology 34, 525-527 (2016).
17 Shannon, P. et al. Cytoscape: a software environment for integrated models of biomolecular interaction networks. Genome research 13, 2498-2504 (2003).
18 Xiangyu Li, K. S., Woonghee Kim, Meng Yuan, Hong Yang, Yusuke Sato, Haruki kume, Seishi Ogawa, Hasan Tuekez, Saeed Shoaie, Jan Boren, Jens Nielsen, Mathias Uhlen, Cheng Zhang, Adil Mardinoglu. Prediction of drug candidates for clear cell renal cell carcinoma using a systems biology- based drug repositioning approach. eBioMedicine (2022).
19 Yuan, M. et al. A Gene Co-Expression Network-Based Drug Repositioning Approach Identifies Candidates for Treatment of Hepatocellular Carcinoma. Cancers 14, 1573 (2022).
20 Shi, B., Ding, J., Qi, J. & Gu, Z. Characteristics and prognostic value of potential dependency genes in clear cell renal cell carcinoma based on a large-scale CRISPR-Cas9 and RNAi screening database DepMap. IntJMed Sci 18, 2063-2075 (2021).
21 Uhlen, M. et al. A pathology atlas of the human cancer transcriptome. Science 357 (2017).
22 Kuleshov, M. V. et al. Enrichr: a comprehensive gene set enrichment analysis web server 2016 update. Nucleic Acids Research 44, W90-W97 (2016).
23 Mering, C. v. et al. STRING: a database of predicted functional associations between proteins. Nucleic Acids Research 31, 258-261 (2003).
24 Han, H. et al. TRRUST v2: an expanded reference database of human and mouse transcriptional regulatory interactions. Nucleic Acids Res 46, D380-d386 (2018).
25 Hanahan, D. & Weinberg, Robert A. Hallmarks of Cancer: The Next Generation. Cell 144, 646-674 (2011).
26 Niess, H. et al. Overexpression of IFN-induced protein with tetratricopeptide repeats 3 (IFIT3) in pancreatic cancer: cellular "pseudoinflammation" contributing to an aggressive phenotype. Oncotarget 6, 3306-3318 (2015).
27 Meyers, R. M. et al. Computational correction of copy number effect improves specificity of CRISPR-Cas9 essentiality screens in cancer cells. Nature Genetics 49, 1779-1784 (2017).
28 Yang, G. H. et al. TCF19 Impacts a Network of Inflammatory and DNA Damage Response Genes in the Pancreatic β-Cell. Metabolites 11, 513 (2021).
29 Efe, J. A. & Ding, S. The evolving biology of small molecules: controlling cell fate and identity. Philosophical Transactions of the Royal Society B: Biological Sciences 366, 2208-2221 (2011).
30 Shimomura, T. et al. MK-5108, a Highly Selective Aurora-A Kinase Inhibitor, Shows Antitumor Activity Alone and in Combination with Docetaxel. Molecular Cancer Therapeutics 9, 157-166 (2010).
31 Giordano, F. et al. p65BTK is a novel potential actionable target in KRAS-mutated/EGFR-wild type lung adenocarcinoma. Journal of Experimental & Clinical Cancer Research 38, 260 (2019).
32 Grimaldi, A. et al. KCa3.1 inhibition switches the phenotype of glioma-infiltrating microglia/macrophages. Cell Death & Disease 7, e2174-e2174 (2016).
33 Subastri, A. et al. Troxerutin with copper generates oxidative stress in cancer cells: Its possible chemotherapeutic mechanism against hepatocellular carcinoma. Journal of Cellular Physiology 233, 1775-1790 (2018).
34 Marxer, M., Ma, H. T., Man, W. Y. & Poon, R. Y. C. p53 deficiency enhances mitotic arrest and slippage induced by pharmacological inhibition of Aurora kinases. Oncogene 33, 3550-3560 (2014).
35 Gunderson, A. J. et al. Bruton Tyrosine Kinase-Dependent Immune Cell Cross-talk Drives Pancreas Cancer. Cancer Discov 6, 270-285 (2016).
36 Guo, R. et al. Regulators CDCA8 as potential targets and biomarkers for the prognosis of human skin cutaneous melanoma. Journal of Cosmetic Dermatology 21, 6034-6048 (2022).
37 Scott, R. E., Ghule, P. N., Stein, J. L. & Stein, G. S. Cell cycle gene expression networks discovered using systems biology: Significance in carcinogenesis. Journal of Cellular Physiology 230, 2533-2542 (2015).
38 de Jong, Y. et al. A screening-based approach identifies cell cycle regulators AURKA, CHK1 and PLK1 as targetable regulators of chondrosarcoma cell survival. Journal of Bone Oncology 19, 100268 (2019).
39 de Groot, C. O. et al. A Cell Biologist's Field Guide to Aurora Kinase Inhibitors. Front Oncol 5, 285 (2015).

## Claims

1. A drug for use in a method of treatment of pancreatic ductal adenocarcinoma (PDAC), wherein the drug is AVL-292 or MK-5108.

2. The drug for use according to claim 1, wherein the method comprises oral administration of the drug.

3. A method of treatment of pancreatic ductal adenocarcinoma (PDAC) in a patient, wherein AVL-292 or MK-5108 is administered to the patient.

4. The method of claim 3, wherein the administration is oral.
